# EUROPÄISCHE PATENTSCHRIFT

(11) **EP 1 765 267 B1**
(45) Veröffentlichungstag und Bekanntmachung des Hinweises auf die Patenterteilung: **06.01.2010**
(21) Anmeldenummer: 05739681.4
(22) Anmeldetag: 12.05.2005
(51) Int. Cl.: A61K 8/41, A61Q 5/10, C07C 205/06, C07C 205/12, C07C 205/19, C07C 205/37, C07C 215/68, C07C 217/84

(54) **PHENYLPROPANDIOL-FARBSTOFFVORPRODUKTE**
INITIAL PHENYLPROPANEDIOL DYE PRODUCTS
PRECURSEURS DE COLORANTS AU PHENYLPROPANEDIOL

(30) Priorität: 30.06.2004 DE 102004031590
(43) Veröffentlichungstag der Anmeldung: 28.03.2007
(73) Patentinhaber: Henkel AG & Co. KGaA, 40589 Düsseldorf (DE)
(72) Erfinder: SÜNGER, Georg, 22081 Hamburg (DE); BRAKE, Carsten, 45481 Mülheim a.d. Ruhr (DE); HÖFFKES, Horst, 40595 Düsseldorf (DE)
(86) Internationale Anmeldenummer: PCT/EP2005/005142
(87) Internationale Veröffentlichungsnummer: WO 2006/002712

(56) Entgegenhaltungen:
- EP-A- 0 999 203
- DE-A1- 2 327 961
- DE-A1- 3 020 236
- DE-A1- 4 219 755
- DE-A1- 10 237 816
- DE-C1- 19 813 937
- JP-A- 63 211 264

## Beschreibung

Die vorliegende Erfindung betrifft Mittel zur Färbung keratinischer Fasern, die spezielle Phenylpropandiol-Derivate enthalten sowie ein Verfahren zur Färbung von Haaren mit diesen Mitteln.

Für das Färben von Keratinfasern, insbesondere menschlichen Haaren, spielen die so genannten Oxidationsfärbemittel wegen ihrer intensiven Farben und guten Echtheitseigenschaften eine bevorzugte Rolle. Solche Färbemittel enthalten Oxidationsfarbstoffvorprodukte, so genannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminöphenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

1-(2,5-Diaminophenyl)ethylenglykol ist als Entwicklersubstanz für Oxidationshaarfärbemittel aus der EP-A-0 999 203 bekannt. Die Herstellung erfolgt ausgehend von 5-Nitroisatin durch Umsetzung mit Natriumhydroxid und nachfolgende Reduktion.

Das Dokument DE-A1-102 37 816 betrifft Haarfärbemittel, die p-Phenylendiaminderivate als Entwickler enthalten können, welche einen an einer Aminogruppe gebundenen Rest -X-C(OR)(OR') tragen können, worin X unter anderem die Bedeutung Methylen aufweisen kann.

Aus Dokument DE-A1-198 13 937 sind oxidative Haarfärbemittel bekannt, die neben einer beliebigen Entwicklersubstanz/Kupplersubstanz-Kombination, zusätzlich mindestens ein Nitrophenylderivat oder Nitropyridinderivat enthalten.

Dokument JP-A-63 211 264 offenbart Phenylpropandiol-Derivate der Formel (I) als Synthesezwischenstufe zur Herstellung von Arzneimitteln und Agrochemikalien.

Aromatische Verbindungen, die Nitrogruppen und Dihydroxyalkylreste aufweisen, sind ebenfalls bekannt. So betrifft EP-A-0 601 138 bzw. DE-A-4 219 755 ein Verfahren zur Herstellung von 5-Alkoxy-2,4-dinitroalkylbenzolen. Der Alkoxyrest kann auch eine oder zwei Hydroxylgruppen zusätzlich tragen. Zudem weist der aromatische Kern einen weiteren Alkylsubstituenten auf.

DT-A-23 27 961 betrifft ein Verfahren zur Herstellung von 2-(o-Nitrophenyl)-1,3-propandiol durch Umsetzung von 2-(o-Nitrophenyl)ethanol mit Formaldehyd unter Zusatz einer starken Base. Anwendungen für das Produkt sind nicht angegeben. DE-A-30 20 236 betrifft ein Verfahren zur Herstellung von o- und p-Nitrophenylalkoholen, die bis zu zwei Nitrogruppen und bis zu drei Hydroxylgruppen enthalten können.

Die vorstehenden 3 Literaturstellen stehen nicht in direktem Bezug zur Haarfärbung.

Gute Oxidationsfarbstoffvorprodukte sollen bei der oxidativen Kupplung die gewünschten Farbnuancen in ausreichender Intensität und Echtheit ausbilden. Sie sollen ferner ein gutes Aufziehvermögen auf die Faser besitzen, wobei insbesondere bei menschlichen Haaren keine merklichen Unterschiede zwischen strapaziertem und frisch nachgewachsenem Haar bestehen sollen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente bzw. Kupplerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, die das vorstehende Eigenschaftsprofil zeigen und die auch in toxikologischer und dermatologischer Hinsicht unproblematisch sind.

Aufgabe der vorliegenden Erfindung war es daher, neue. Farbstoffvorprodukte bzw. Oxidationsfarbstoffvorprodukte, insbesondere Kupplerkomponenten und Entwicklerkomponenten zu entwickeln, die die an Oxidationsfarbstoffvorprodukte gestellten Anforderungen, auch hinsichtlich der toxikologischen und dermatologischen Eigenschaften, erfüllen und Färbungen in einem breiten Farbspektrum mit guten Echtheitseigenschaften ermöglichen.

Es wurde erfindungsgemäß gefunden, dass spezielle Phenylpropandiol-Derivate, insbesondere 2-(Diaminophenyl)propan-1,3-diol-Derivate, den an Oxidationsfarbstoffvorprodukte, insbesondere an Entwickler- und Kupplerkomponenten gestellten Anforderungen in einem hohen Maße genügen. Sie erlauben insbesondere hohe Farbintensitäten, eine gute Egalisierung sowie gute Echtheiten.

Ein erster Gegenstand der Erfindung sind daher Mittel zur Färbung keratinischer Fasern, insbesondere menschlicher Haare, enthaltend in einem kosmetisch akzeptablen Träger als Farbstoffvorprodukt mindestens ein Phenylpropandiol-Derivat der allgemeinen Formel (I) 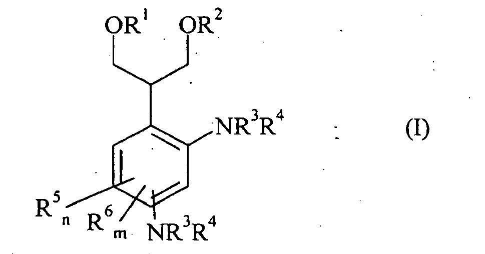 mit der Bedeutung
- R¹, R², R³, R⁴: unabhängig voneinander H, C₁₋₆-Alkyl, C₁₋₆- Hydroxyalkyl, C₂₋₆-Dihydroxyalkyl, C₇₋₁₂-Arylalkyl, C₂₋₆- Polyhydroxyalkyl, C₂₋₆-Alkoxyalkyl, C₁₋₆-Aminoalkyl, C₂₋ ₁₀-Alkylaminoalkyl, C₃₋₁₂-Dialkylaminoalkyl, wobei R¹ und R² auch gemeinsam einen C₁₋₃-Alkylenrest bilden können, der durch Halogen, Hydroxy oder C₁₋₄-Alkyl substituiert sein kann, Halogenalkyl, gegebenenfalls substituierter aromatischer Rest, C₂₋₆-Alkenyl,
- R⁵: unabhängig voneinander H, OH, Halogen, NH₂, C₁₋₆- Alkylamino, C₂₋₁₂-Dialkylamino, NO₂, COOH, CN, C₁₋₆- Alkyl, C₁₋₆-Hydroxyalkyl, C₂₋₆-Polyhydroxyalkyl, C₇₋₁₂- Arylalkyl, C₂₋₆-Alkoxyalkyl, C₁₋₆-Aminoalkyl, C₂₋₁₀- Alkylaminoalkyl, C₃₋₁₂-Dialkylaminoalkyl, C₁₋₆-Alkoxy, gegebenenfalls substituierter aromatischer Rest, C₂₋₆- Alkenyl,
- R⁶: H, Halogen, C₁₋₆-Alkoxy, C₁₋₆-Alkylthio,
- m: 0 oder 1,
- n: ganze Zahl von 0 bis (3-m),
ausgewählt aus Phenylpropandiol-Derivaten der allgemeinen Formeln (la) bis (Id)

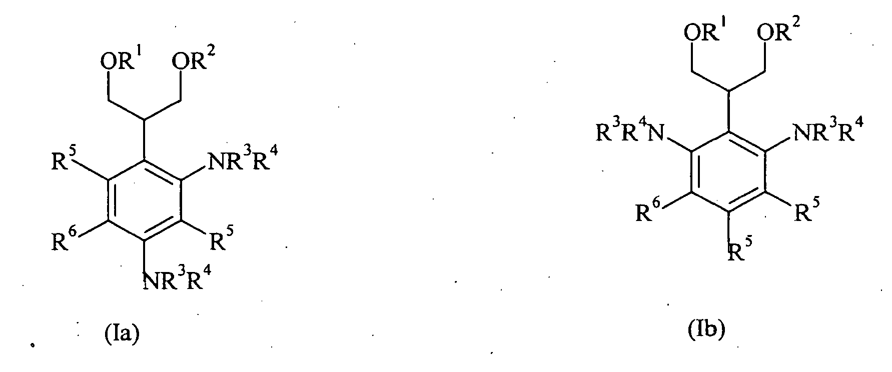

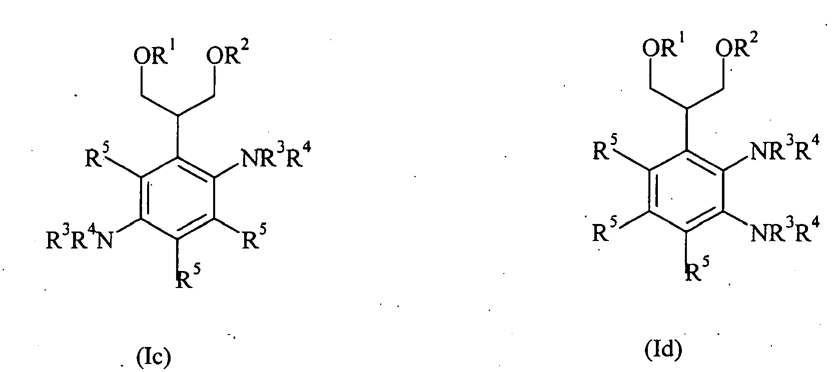

Die Erfindung betrifft insbesondere Mittel zur oxidativen Färbung keratinischer Fasern, die mindestens ein Farbstoffvorprodukt der allgemeinen Formel (I) enthalten. Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die Farbstoffvorprodukte der allgemeinen Formel (I) sind gekennzeichnet durch einen von Propandiol abgeleiteten Rest und zwei von Aminogruppen abgeleitete Reste, die an einen Benzolkern kovalent gebunden sind. Eine Aminogruppe liegt dabei in o-Stellung zur Propandiolgruppe vor.

Abhängig von der Stellung der zweiten Aminogruppe handelt es sich bei den Verbindungen der allgemeinen Formel (I) um Kupplerkomponenten oder Entwicklerkomponenten, die in der oxidativen Haarfärbung eingesetzt werden. Die Verbindungen der allgemeinen Formeln (la) und (Ib) sind Kupplerkomponenten, während die Verbindungen der allgemeinen Formeln (Ic) und (Id) Entwicklerkomponenten sind. Die Kupplerkomponenten weisen mindestens eine Position am Benzolkern auf, an dem die Kupplung erfolgen kann. Diese Position ist beispielsweise durch den Rest R⁶ definiert, da R⁶ Wasserstoff oder eine Abgangsgruppe, ausgewählt aus Halogen, C₁₋₆-Alkoxy und C₁₋₆-Alkylthio definiert. Sofern einer oder mehrere der Reste R⁵ ebenfalls Wasserstoff oder eine Abgangsgruppe bedeuten, kann auch an diesen Positionen eine Kupplung erfolgen. In den Entwicklerkomponenten ist keine derartige Position am Benzolkern notwendig, so dass alle Reste R⁵ auch von Wasserstoff oder Abgangsgruppen verschieden sein können. Kupplerkomponenten und Entwicklerkomponenten verbindet das Vorliegen zweier Aminogruppen und einer Propandiolgruppe bzw. von diesen abgeleiteter Gruppen, wobei eine der Aminogruppen in o-Stellung zur Propandiolgruppe vorliegt. Jeder der angegebenen Reste kann in jeder der angegebenen Positionen eine unterschiedliche Bedeutung aufweisen. Es ist jedoch auch möglich, dass die Reste gleiche Bedeutungen haben. In den Verbindungen der allgemeinen Formel (I) liegen vorzugsweise ein oder zwei von Wasserstoff verschiedene Reste R⁵ bzw. R⁶ vor. Besonders bevorzugt liegt ein von Wasserstoff verschiedener Rest R⁵ bzw. R⁶ vor.

Gemäß einer bevorzugten Ausführungsform weist das Farbstoffvorprodukt die allgemeine Formel (Ie) auf

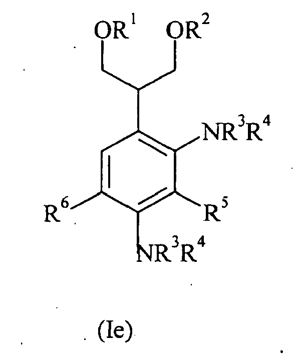

Dabei ist besonders bevorzugt maximal einer der Reste R⁵ und R⁶ von Wasserstoff verschieden. R¹ bis R⁴ sind vorzugsweise an allen Positionen Wasserstoff. R⁶ ist besonders bevorzugt ein C₁₋₄-Alkoxyrest, insbesondere ein Methoxyrest oder Ethoxyrest, oder R⁶ ist Wasserstoff.

R⁵ ist besonders bevorzugt Wasserstoff oder ein C₁₋₄-Hydroxyalkylrest, speziell ein Rest-CH₂CH₂OH.

Da es sich bei den erfindungsgemäßen Derivaten um Amino-Verbindungen handelt, lassen sich aus diesen in üblicher Weise die bekannten Säureadditionssalze herstellen. Die Erfindung bezieht sich daher sowohl auf die in freier Form vorliegenden Verbindungen als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₆-Alkylgruppen, vorzugsweise C₁-C₄-Alkylgruppen, die linear oder verzweigt sein können, sind die Gruppen Methyl, Ethyl, Propyl, Isopropyl, Butyl, Pentyl und Hexyl. Ethyl und Methyl sind bevorzugte Alkylgruppen. Als bevorzugte C₁- bis C₆-(Mono)hydroxyalkylgruppe können eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. C₂₋₆-Alkoxyalkylreste weisen insgesamt 2 bis 6 C-Atome auf, die sich auf den Alkoxy- und den Alkylrest verteilen. Es können damit im Alkoxy- bzw. Alkylrest jeweils bis zu 5 C-Atome vorliegen. Bevorzugt handelt es sich um einen C₁₋₃-Alkoxy-C₁₋₃-Alkylrest, besonders bevorzugt C₁₋₂-Alkoxy-C₁₋₂-Alkylrest. Sowohl der Alkoxy- als auch der Alkylrest können linear oder verzweigt sein. Sie sind vorzugsweise linear. C₁₋₆-Aminoalkylreste, vorzugsweise C₁₋₄-Aminoalkylreste, C₂₋₁₀-Alkylaminoalkylreste, vorzugsweise C₂₋₆-Alkylaminoalkylreste und C₃₋₁₂-Dialkylaminoalkylreste, vorzugsweise C₃₋₉-Dialkylaminoalkylreste können jeweils lineare oder verzweigte Alkylreste aufweisen. Jede einzelne Alkylgruppe weist dabei vorzugsweise 1 bis 3 C-Atome auf. Die in den jeweiligen Gruppen vorliegenden Alkylreste sind vorzugsweise wie vorstehend bei den reinen Alkylresten definiert. Arylalkylreste sind bevorzugt Phenyl-C₁₋₄-Alkylreste. Halogen bedeutet vorzugsweise Fluor, Chlor oder Brom, insbesondere Chlor. C₁₋₆-Alkylthio ist bevorzugt C₁₋₄-Alkylthio, insbesondere Methylthio. C₁₋₆-Alkylamino ist besonders bevorzugt C₁₋₄-Alkylamino, insbesondere Methylamino oder Ethylamino. C₂₋₁₂-Dialkylamino ist bevorzugt Di-C₁₋₄-Alkylamino, insbesondere Dimethylamino oder Diethylamino.

Sofern R¹ und R² gemeinsam einen C₁₋₃-Alkylenrest bilden, handelt es sich bevorzugt um einen Methylenrest oder Ethylenrest, speziell einen Methylenrest. Dieser kann wie angegeben substituiert sein, ist jedoch bevorzugt nicht substituiert.

Besonders bevorzugt weist ein Derivat der allgemeinen Formel (I) insgesamt maximal vier OH-Gruppen, vorzugsweise maximal drei OH-Gruppen auf. Es liegen im Derivat der allgemeinen Formel (I) vorzugsweise maximal vier Stickstoffatome besonders bevorzugt zwei Stickstoffatome vor. Insgesamt liegen, abgesehen von den Hydroxylgruppen, im Derivat der allgemeinen Formel (I) maximal weitere vier Sauerstoffatome, vorzugsweise maximal weitere zwei Sauerstoffatome, insbesondere maximal ein weiteres Sauerstoffatom vor.

Bevorzugte Bedeutungen der Reste werden nachstehend aufgeführt.

Gemäß einer bevorzugten Ausführungsform sind R¹, R², R³, R⁴ unabhängig voneinander Wasserstoff, C₁₋₄-Alkyl oder C₁₋₄-Hydroxyalkyl. Dabei bedeuten besonders bevorzugt R¹ und R² Wasserstoff und R³ und R⁴ Wasserstoff oder C₁₋₄-Hydroxyalkyl.

Besonders bevorzugt bedeuten R¹ bis R⁴ Wasserstoff.

Gemäß einer Ausführungsform der Erfindung bedeutet R⁵ unabhängig voneinander Wasserstoff, C₁₋₄-Alkyl oder C₁-₄-Hydroxyalkyl.

Besonders bevorzugt bedeutet m 0 oder 1.

Gemäß einer weiteren besonders bevorzugten Ausführungsform der Erfindung bedeutet R⁶ Wasserstoff oder C₁₋₄-Alkoxy.

Das erfindungsgemäße Mittel kann ein oder mehrere, beispielsweise mindestens zwei unterschiedliche Derivate der allgemeinen Formel (I) enthalten.

Die erfindungsgemäßen Mittel können sowohl Kuppler als auch Entwickler enthalten, die der allgemeinen Formel (I) entsprechen. Bevorzugt enthalten die Mittel entweder Entwickler oder Kuppler, die der allgemeinen Formel (I) entsprechen. Besonders bevorzugt sind Kuppler der allgemeinen Formeln (la), (Ib) und (Ie).

Die erfindungsgemäßen Kuppler ergeben sehr intensive Färbungen im Blaubereich, die sich durch gute Echtheiten auszeichnen.

Die Derivate der Formel (I) lassen sich mit Hilfe herkömmlicher organischer Methoden herstellen. Beispielhaft sei an dieser Stelle auf die Versuchsdurchführungen im Rahmen der Ausführungsbeispiele verwiesen.

Die erfindungsgemäßen Mittel zur, insbesondere oxidativen, Färbung keratinischer Fasern enthalten mindestens ein Derivat der allgemeinen Formel (I) vorzugsweise in einer Menge von 0,01 bis 10 Gew.-%, besonders bevorzugt 0,1 bis 3 Gew.-%, bezogen auf das fertige Mittel.

Das Mittel zur Färbung keratinischer Fasern kann dabei aus beliebigen geeigneten Mitteln ausgewählt werden, die insbesondere zur Färbung menschlicher Haare geeignet sind. Als kosmetisch akzeptabler Träger wird dabei insbesondere ein ansonsten üblicher Träger von Mitteln zur Färbung menschlicher Haare eingesetzt. Die erfindungsgemäßen Färbemittel können dabei, abgesehen von den Derivaten der allgemeinen Formel (I) entsprechend bekannter Färbemittel zusammengesetzt sein bzw. die für diese üblichen inhaltsstoffe enthalten. Beispiele weiterer geeigneter und erfindungsgemäß bevorzugter Inhaltsstoffe sind nachstehend angegeben.

Eine allgemeine Zusammensetzung ist nachstehend angegeben:

| | |
|---|---|
| Entwickler | 0,05-5% |
| Kuppler | 0,05-5% |
| Tenside, Emulgatoren | 0,1 - 20 % |
| Fettalkohole und andere | 0,5-20% |
| Emulsionsbildner | |
| Komplexierungsmittel | 0,05-10% |
| Puffermittel | 0,1-1,0% |
| Löslichkeitsvermittler + Lösungsmittel | 0,5 - 15% |
| pH-Stellmittel | Nach Bedarf |
| Parfümöle | 0,1 - 0,6% |
| Polymere | 0,1 - 5% |
| Wasser | 50 - 98% |

Geeignete Färbemittel-Zusammensetzungen sind beispielsweise in DE-U1-299 11 819, DE-A-101 25 451, DE-U1-201 11 036, Kosmetik, Hrsg. W. Umbach, 2. Aufl. 1995, G. Thieme Verlag Stuttgart, New York beschrieben.

Neben den Derivaten der Formel (I) können die erfindungsgemäßen Färbemittel ferner mindestens eine weitere Entwicklerkomponente enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente (zusätzlich) ein p-Phenylendiaminderivat oder eines seiner physiologisch verträglichen Salze einzusetzen.

Erfindungsgemäß ganz besonders bevorzugt eingesetzte p-Phenylendiaminderivate sind p-Phenylendiamin, p-Toluylendianiin, 2-(β-Hydroxyethyl)-p-phenylendiamin, 2-(α,β-Dihydroxyethyl)-p-phenylendiamin und N,N-Bis-β-hydroxyethyl)-p-phenylendiamin.

Es kann erfindungsgemäß weiterhin bevorzugt sein, als Entwicklerkomponente (zusätzlich) Verbindungen einzusetzen, die mindestens zwei aromatische Kerne enthalten, die mit Amino- und/oder Hydroxylgruppen substituiert sind.

Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch erträglichen Salze.

Weiterhin kann es erfindungsgemäß bevorzugt sein, als (zusätzliche) Entwicklerkomponente ein p-Aminophenolderivat oder eines seiner physiologisch verträglichen Salze einzusetzen.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die (zusätzliche) Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die (zusätzliche) Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, . 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

In einer weiteren bevorzugten Ausführungsform enthalten die erfindungsgemäßen Färbemittel mindestens eine bzw. mindestens eine weitere Kupplerkomponente.

Als (weitere) Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß besonders bevorzugte (weitere) Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

In einer weiteren Ausführungsform der vorliegenden Erfindung können die Färbemittel mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den erfindungsgemäßen Derivaten der Formel (I) können die erfindungsgemäßen Färbemittel in einer weiteren bevorzugten Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

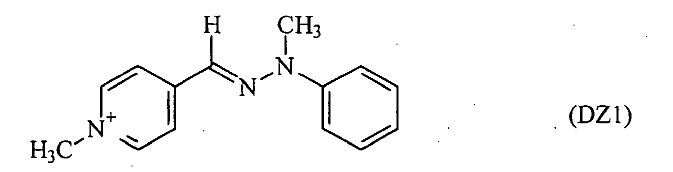

CH₃SO₄

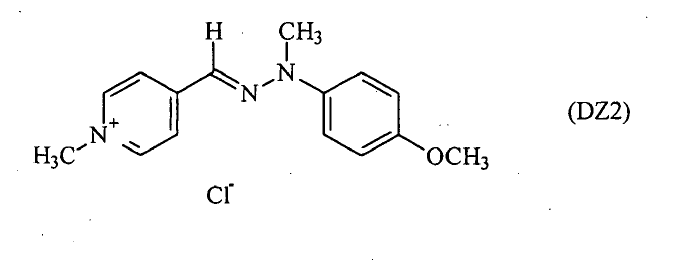

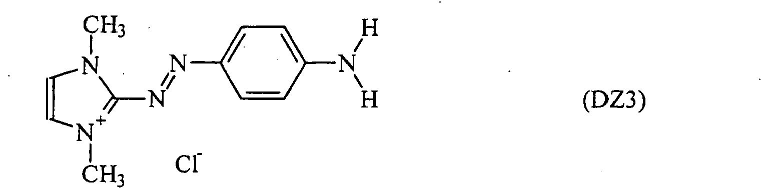

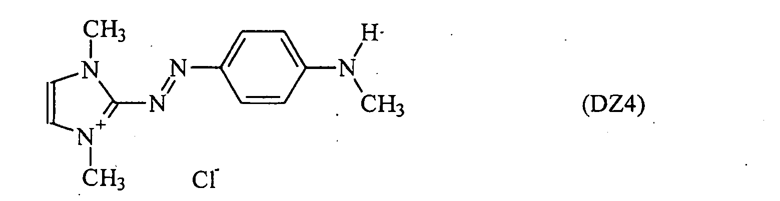

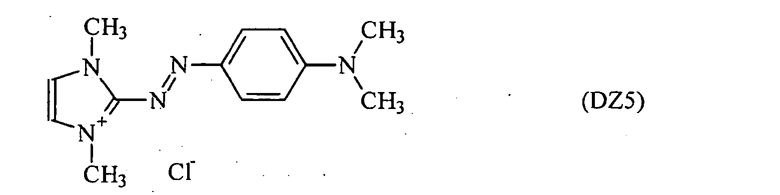

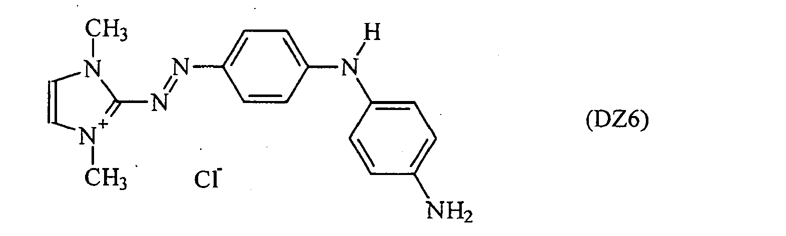

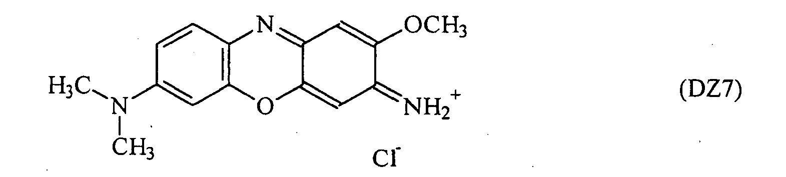

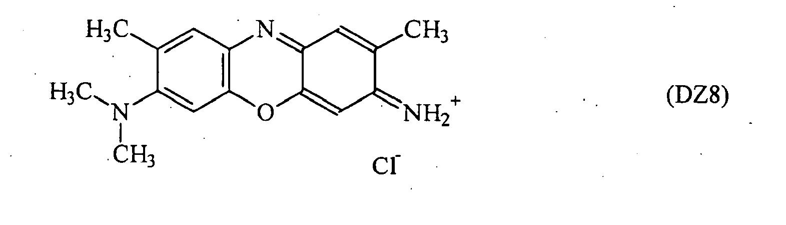

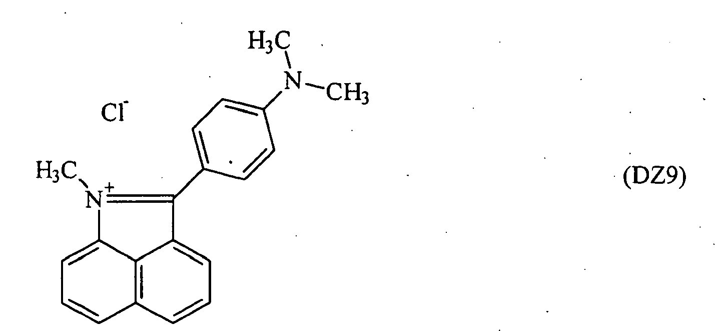

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter der Marke Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmächende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beilspielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "OxoAlkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Besonders bevorzugte ampholytische Tenside sind das N-Kokosalkylaminopropionat, das Kokosacylaminoethylaminopropionat und das C₁₂₋₁₈-Acylsarcosin. Erfindungsgemäß können als kationische Tenside insbesondere solche vom Typ der quartären Ammoniumverbindungen, der Esterquats und der Amidoamine eingesetzt werden.

Bei Esterquats handelt es sich um bekannte Stoffe, die sowohl mindestens eine Esterfunktion als auch mindestens eine quartäre Ammoniumgruppe als Strukturelement enthalten.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren. Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Anmeldung ist die Verwendung der erfindungsgemäßen Derivate zum Färben keratinischer Fasern.

Ein dritter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Ein fünfter Gegenstand der vorliegenden Erfindung ist eine Synthesezwischenstufe, ausgewählt aus der Gruppe, gebildet von 2-(5-Ethoxy-2,4-dinitrophenyl)propan-1,3-diol, 2-(5-Methoxy-2,4-dinitrophenyl)propan-1,3-diol, 2-(3-(2-Hydroxyethyl)-2,4-dinitrophenyl]propan-1,3-diol.

Die Erfindung wird durch die nachstehenden Beispiele näher erläutert.

### Synthesebeispiele:

1. 2-(2,4-Diamino-3-(2-hydroxyethyl)phenyl]propan-1,3-diol, Dihydrochlorid Die Substanz wurde analog der folgenden dreistufigen Reaktionssequenz ausgehend von 2-Nitro-m-xylol dargestellt: 1. Stufe: 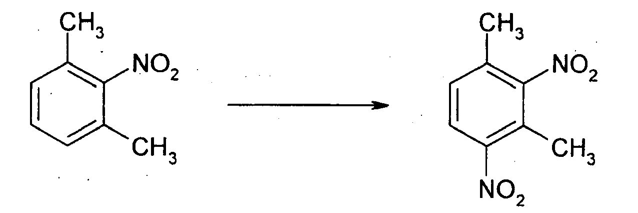
2. Stufe: 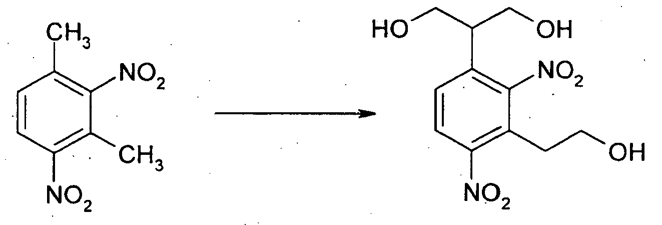
3. Stufe:

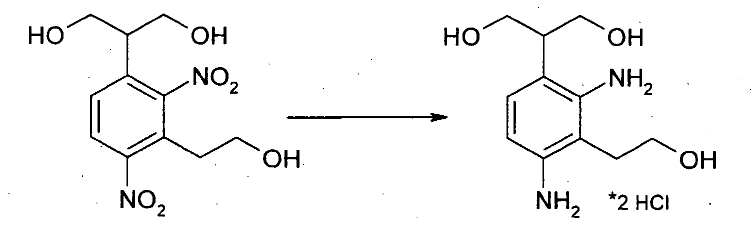

1. Stufe: Darstellung von 2,4-Dimethyl-1,3-dinitrobenzol
   In 73 ml Schwefelsäure (konz.) wurden unter Kühlung bei 15°C 25 g (165 mmol) 2-Nitro-m-xylol gelöst. Zu der resultierenden dunkelgelben, klaren Lösung wurde anschließend bei 15°C vorsichtig ein Gemisch aus 11,9 ml Salpetersäure (konz.) und 18,3 ml Schwefelsäure (konz.) getropft. Man erhielt eine gelbe, schaumige Suspension, die für eine Stunde bei Raumtemperatur gerührt wurde. Anschließend goss man das Reaktionsgemisch auf ca. 210 g Eis, filtrierte den Niederschlag ab und wusch diesen bis zur Neutralität mit reichlich Wasser. Das erhaltene Rohprodukt wurde im Vakuum getrocknet und danach aus Ethanol umkristallisiert. Man erhielt weiß-gelbe Kristalle, die im Vakuum bei 50°C getrocknet wurden.
   Ausbeute: 28,1 g (87%)
   Schmp.: 84°C
   ¹H-NMR (400 MHz, CDCl₃): δ = 2,35 (s, 3H); 2,45 (s, 3H); 7,30 (d, 1H), 7,90 (d, 1H).
2. Stufe: Darstellung von 2-[3-(2-Hydroxyethyl)-2,4-dinitrophenyl]propan-1,3-diol In 160 ml DMSO wurden 16 g (81,5 mmol) 2,4-Dimethyl-1,3-dinitrobenzol aus Stufe 1 und 27,2 ml Formalin (35 %ig) vorgelegt. Zu dieser Lösung wurden 0,9 g (16 mmol) Kaliumhydroxid gelöst in 7 ml Formalin (35 % ig) zugetropft. Das Reaktionsgemisch wurde für 30 Minuten gerührt, wobei die einsetzende, zunächst leicht exotherme Reaktion durch Eiskühlung auf Raumtemperatur gehalten wurde. Anschließend wurde auf 480 ml Wasser gegossen, mit wenig Salzsäure neutralisiert und sechsmal mit jeweils 250 ml Ether extrahiert. Die vereinigten Etherphasen wurden mit Natriumsulfat getrocknet, filtriert und im Vakuum bis zur Trockene eingeengt. Es resultierte ein gelbes Öl, welches im Vakuum getrocknet wurde.
   Ausbeute: 11,6 g (50%)
   - ¹H-NMR (400 MHz, DMSO-d₆):: δ = 2,75 (m, 1H); 2,85 (t, 2H); 3,60 (t, 2H); 3,70 (m, 4H); 4,80 (t, 2H,OH); 4,90 (t, 1H, OH) 7,70 (d, 1H); 8,10 (d, 1H).
3. Stufe: Darstellung von 2-[2,4-Diamino-3-(2-hydroxyethyl)phenyl]propan-1,3-diol,
   Dihydrochlorid
   11,6 g (40,5 mmol) 2-[3-(2-hydroxyethyl)-2,4-dinitrophenyl]propan-1,3-diol aus Stufe 2 wurden in 450 ml Ethanol und 50 ml Wasser in Gegenwart von 0,5 g Katalysator (Pd/C) bei 50°C und 50 bar Wasserstoff im Autoklaven hydriert. Nach beendeter Wasserstoffaufnahme wurde die erhaltene Lösung mit Salzsäure (10%ig) angesäuert, vom Katalysator abfiltriert und zur Trockene eingeengt. Man erhielt ein grau-braunes Pulver, welches im Vakuum getrocknet wurde.
   Ausbeute: 7,6 g = 63%,
   ¹H-NMR (400 MHz, D₂O): δ = 2,95 (m, 2H); 3,25 (m, 1H); 3,70-3,95 (m, 6H); 7,00 (d, 1H); 7,30 (d, 1H)

### 11. 2-(2,4-Diamino-5-ethoxyphenyl)propan-1,3-diol, Dihydrochlorid

Die Substanz wurde analog der folgenden vierstufigen Reaktionssequenz ausgehend von 3-Fluortoluol dargestellt: 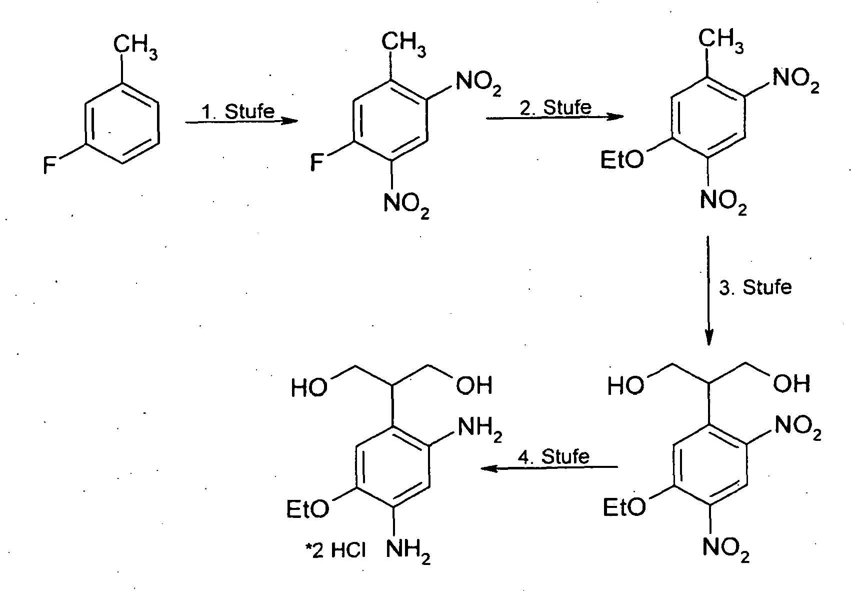
1. Stufe: Synthese von 5-Fluor-1-methyl-2,4-dinitrobenzol gemäß
   Egawa et. al., J. Heterocycl. Chem. 1987, 24, 181-185.
2. Stufe: Darstellung des 5-Ethoxy-1-methyl-2,4-dinitrobenzol gemäß
   DE 4219755 A1, Wella AG.
3. Stufe: Darstellung von 2-(5-Ethoxy-2,4-dinitrophenyl)propan-1,3-diol
   In 275 ml DMSO wurden 31 g (137 mmol) 5-Ethoxy-1-methyl-2,4-dinitrobenzol aus Stufe 2 und 29 ml Formalin (35 %ig) vorgelegt. Zu dieser Lösung wurden 2 g (13 mmol) 1,8-Diazabicyclo[5.4.0]undec-7-en gegeben.
   Das Reaktionsgemisch wurde für zwei Stunden bei Raumtemperatur gerührt und dann auf 800 ml Wasser gegeben, mit wenig Salzsäure neutralisiert und sechsmal mit jeweils 200 ml Ether extrahiert. Die vereinigten Etherphasen wurden mit Natriumsulfat getrocknet, filtriert und im Vakuum bis zur Trockene eingeengt. Das zunächst resultierende Öl wurde aus wenig Essigsäure-ethylester kristallisiert. Man erhielt einen weißen Feststoff.
   Ausbeute: 13,2 g (34%)
   Schmp.: 88 °C
   - ¹H-NMR (400 MHz, DMSO-d₆):: δ = 1,35 (t, 3H); 3,35 (m, 1H); 3,75 (m, 4H); 4,35 (q, 2H); 4,80 (t, 2H, OH); 7,40 (s, 1H); 8,45 (s, 1H).
4. Stufe: Darstellung von 2-(2,4-Diamino-5-ethoxyphenyl)propan-1,3-diol, Dihydrochlorid
   13,1g (45,8 mmol) 2-(5-Ethoxy-2,4-dinitrophenyl)propan-1,3-diol aus Stufe 3 werden in 450 ml Ethanol und 50 ml Wasser in Gegenwart von 0,5 g Katalysator bei Raumtemperatur unter Normaldruck hydriert. Nach beendeter Wasserstoffaufnahme wurde die erhaltene Lösung mit Salzsäure (10%ig) angesäuert, vom Katalysator abfiltriert und zur Trockene eingeengt. Man erhielt einen hellen, grau-braunen Feststoff.
   Ausbeute: 11,4 g = 83%
   - ¹H-NMR (400 MHz, D₂O):: δ = 1,45 (t, 3H); 3,35 (m, 1H); 3,75 (m, 2H); 3,95 (m, 2H); 4,20 (q, 2H); 7,10 (s, 1H); 7,20 (s, 1H).

### III. 2-(2,4-Diamino-5-methoxyphenyl)propan-1,3-diol, Dihydrochlorid

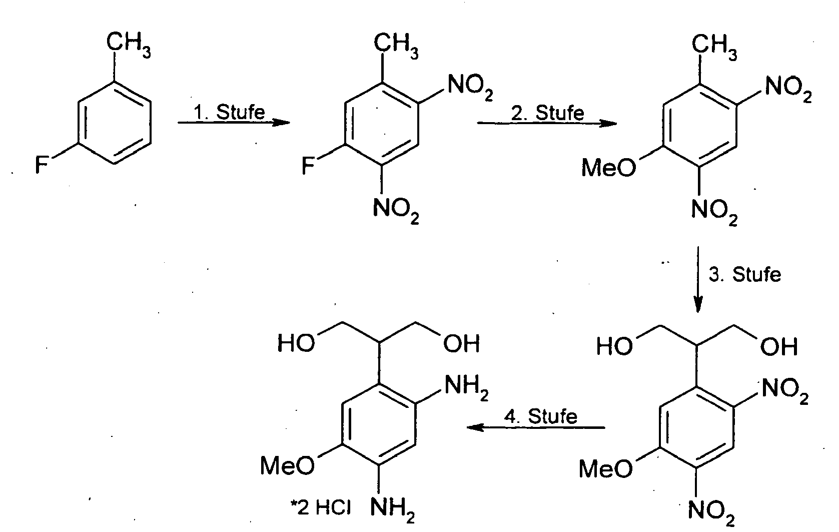

Die Substanz wurde analog der folgenden vierstufigen Reaktionssequenz ausgehend von 3-Fluortoluol dargestellt:
1. Stufe: Synthese von 5-Fluor-1-methyl-2,4-dinitrobenzol gemäß Literaturvorschrift:
   Egawa et. al., J. Heterocycl. Chem. 1987, 24, 181-185.
2. Stufe: Darstellung des 5-Methoxy-1-methyl-2,4-dinitrobenzol gemäß Patentschrift: DE 4219755 A1, Wella AG.
3. Stufe: Darstellung von 2-(5-Methoxy-2,4-dinitrophenyl)propan-1,3-diol
   In 140 ml DMSO wurden 15 g (137 mmol) 5-Methoxy-1-methyl-2,4-dinitrobenzol aus Stufe 2 und 15,8g Formalin (35 %ig) vorgelegt. Zu dieser Lösung werden 1 g (6,5 mmol) 1,8-Diazabicyclo[5.4.0]undec-7-en gegeben.
   Das Reaktionsgemisch wurde für drei Stunden bei Raumtemperatur gerührt und dann auf 400 ml Wasser gegeben, mit wenig Salzsäure neutralisiert und dreimal mit jeweils 300 ml Ether extrahiert. Die vereinigten Etherphasen wurden mit Natriumsulfat getrocknet, filtriert und im Vakuum bis zur Trockene eingeengt. Man erhielt ein gelbes Öl.
   Ausbeute: 14,8 g (86%)
   1H-NMR (400 MHz, DMSO-d6): δ = 3,55 (m, 1H); 3,75 (m, 4H); 4,05 (s, 3H); 4,80 (t, 2H, OH); 7,40 (s, 1H); 8,50 (s, 1H).
4. Stufe: Darstellung von 2-(2,4-Diamino-5-methoxyphenyl)propan-1,3-diol, Dihydrochlorid
   14,4g (59,4 mmol) 2-(5-Methoxy-2,4-dinitrophenyl)propan-1,3-diol aus Stufe 3 wurden in 250 ml Methanol in Gegenwart von 0,3 g Katalysator bei Raumtemperatur unter Normaldruck hydriert. Nach beendeter Wasserstoffaufnahme wurde die erhaltene Lösung mit Salzsäure (10%ig) angesäuert, vom Katalysator abfiltriert und zur Trockene eingeengt. Man erhielt einen hellen, grau-braunen Feststoff.
   Ausbeute: 11,4 g = 67 %
   1H-NMR (400 MHz, D2O): δ = 3,35 (m, 1H); 3,85 (m, 2H); 4,05 (m, 5H); 7,20 (s, 1H); 7,45 (s, 1H).

### IV. 2-(2,5-Diamino-4-methoxyphenyl)propan-1,3-diol, Dihydrochlorid

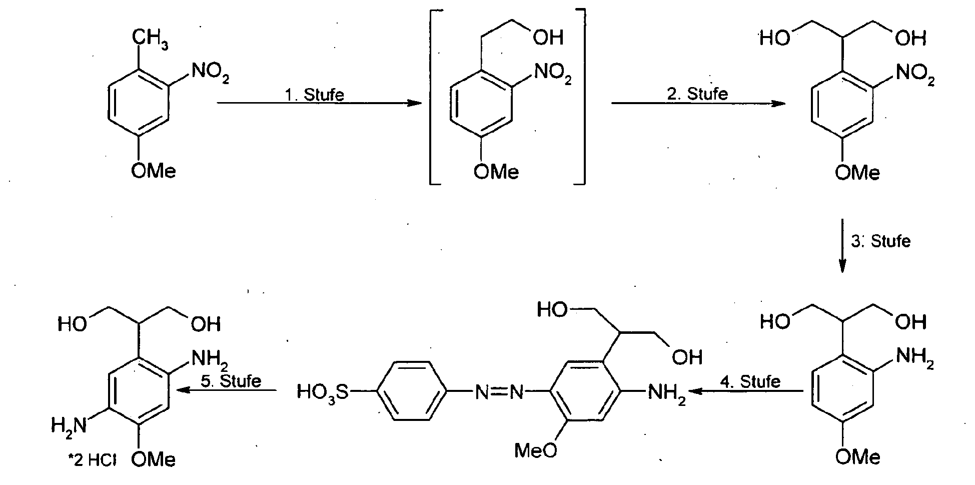

Die Substanz wurde analog der folgenden fünfstufigen Reaktionssequenz ausgehend von 4-Methyl-3-nitroanisol dargestellt:

### 1. Stufe & 2. Stufe:

Synthese von 2-(4-Methoxy-2-nitrophenyl)ethanol & Darstellung des 2-(4-Methoxy-2-nitrophenyl)propan-1,3-diol:

Zu einer Lösung von 26,7g (160mmol) 4-Methyl-3-nitroanisol und 27,6g (873mmol) p-Formaldehyd in 300mL DMSO, wurden unter Rühren bei Raumtemperatur 1,7g (30,3mmol) KOH-Pulver zugegeben. Die resultierende gelbe Suspension wurde für 24 Stunden bei Raumtemperatur gerührt. Danach wurde die Reaktionslösung auf 600mL Wasser gegeben, mit wenig verdünnter Salzsäure neutralisiert und auf 4°C abgekühlt. Der ausgefallene Niederschlag wurde abfiltriert und verworfen. Die verbliebene Mutterlauge wurde mit Natriumchlorid gesättigt und dann sechsmal mit je 400mL MTBE extrahiert.

Die vereinigten MTBE-Phasen wurden mit Natriumsulfat getrocknet und am Rotationsverdampfer komplett eingeengt.

Der Rückstand wurde im Ölpumpenvakuum bei 50°C getrocknet und dann im Kugelrohr destilliert (Pumpe: IImvac, 1 *10⁻⁵mbar). Man erhielt zwei Fraktionen:
1. Fraktion, <180°C: 4-Methyl-3-nitroanisol
2. Fraktion, 180-214°C: 24,0 g Gemisch aus 2-(4-Methoxy-2-nitrophenyl)ethanol & 2-(4-Methoxy-2-nitrophenyl)propan-1,3-diol (orange-gelbes Öl)
   Sumpf: 3,3 g = 9% 2-(4-Methoxy-2-nitrophenyl)propan-1,3-diol (gelber Feststoff;
   Schmp.: 67-72°C)

Die 24,0g der zweiten Fraktion wurden gemeinsam mit 14,2g (449 mmol) p-Formaldehyd in 200mL DMSO gelöst. Unter Rühren wurden 1,1 g (19,6 mmol) KOH-Pulver zugesetzt und für 23 Stunden bei RT gerührt. Anschließend wurde die Reaktionslösung auf 600mL Wasser gegeben, mit wenig verd. Salzsäure neutralisiert und mit Natriumchlorid gesättigt. Die erhaltene Lösung wurde dreimal mit je 400mL MTBE extrahiert, die vereinigten MTBE-Phasen mit Natriumsulfat getrocknet und am Rotationsverdampfer eingeengt. Der erhaltene Rückstand wurde dann im Kugelrohr destilliert (Pumpe: IImvac, 1*10⁻⁵mbar), wobei bis 190°C verbliebene Verunreinigungen abdestilliert wurden. Es verblieben 15,5g = 42,3 % reinen Produktes als Destillationssumpf zurück.
Gesamtausbeute: 18,8g = 52% gelber Feststoff; 67-72°C
¹H-NMR (400 MHz, DMSO-d₆): δ = 3,15 (m, 1H); 3,60 (m, 2H); 3,70 (m, 2H); 3,82 (s, 3H); 4,68 (t, 2H, OH); 7,18 (dd, 1H); 7,30 (d, 1H); 7,45 (d, 1H).

### 3. Stufe: Darstellung von 2-(2-Amino-4-methoxyphenyl)propane-1,3-diol

19,1 g (84,1mmol) des 2-(4-Methoxy-2-nitrophenyl)propan-1,3-diol aus Stufe 2 wurden in 400 ml Methanol in Gegenwart von 0,5 g Katalysator (Pd 5% auf C) bei Raumtemperatur unter Normaldruck hydriert. Nach beendeter Wasserstoffaufnahme wurde die erhaltene Lösung vom Katalysator abfiltriert, fast zur Trockene eingeengt und bei 4°C gelagert. Der ausgefallene Niederschlag wurde abfiltriert, mit wenig Methanol gewaschen und dann bei 50°C im Vakuum getrocknet.
Ausbeute: 11,4g = 69%, gelber Feststoff (Schmp.: 98-103)
¹H-NMR (400 MHz, DMSO-d₆): δ = 2,80 (m, 1H); 3,50 (m, 2H); 3,65 (m, 5H); 4,50 (t, 2H, OH); 4,80 (s, 2H, NH₂); 6,10 (dd, 1H); 6,20 (d, 1H); 6,80 (d, 1H).

### 4. Stufe: Darstellung von 4-({4-Amino-5-[2-hydroxy-l-(hydroxymethyl)ethyl]-2-methoxyphenyl}diazenyl)benzolsulfonsäure

Zur Herstellung der Diazoniumchlorid-Lsg wurden 16,3g (94,1 mmol) Sulfanilsäure in 47mL (2M) Natronlauge gegeben. Zu dieser Suspension wurde eine Lösung von 6,5g (94,2mmol) Natriumnitrit in 90mL Wasser gegeben und man erhielt eine hell-orarige-farbene Lösung. Diese wurde auf 0-5°C gekühlt (Eis/-Kochsalz-Mischung) und mit 81mL 10%ige Salzsäure versetzt. Die erhaltene hell-orangefarbene Diazonium-chlorid-Suspension wurde auf 0-5°C abgekühlt und wie im Folgenden beschrieben verwendet.

Zu 17,1 g (86,7mmol) 2-(2-amino-4-methoxyphenyl)propane-1,3-diol wurden 87mL 1 molare Salzsäure gegeben. Die resultierende Emulsion wurde ebenfalls auf 5-10°C gekühlt und dann langsam mit der oben beschriebenen Diazoniumchlorid-Suspension versetzt. Die Reaktionslösung verfärbte sich während des Rührens schon nach kurzer Zeit rot. Nach 5 Stunden rühren bei 5-10°C wurde die Temperatur langsam auf Raumtemperatur erhöht. Anschließend wurde mit Natronlauge (10%ig) neutralisiert, die Reaktionslösung am Rotationsverdampfer komplett eingeengt und der Rückstand in 150mL Methanol aufgeschlämmt und filtriert. Danach wurde die verbliebene Lösung noch zweimal mit je 50mL Methanol gewaschen und die komplette Methanollösung am Rotationsverdampfer eingeengt. Der orange rote Rückstand wurde bei 50°C im Vakuum getrocknet.
Ausbeute: 23,4g =71% orangeroter Feststoff
¹H-NMR (400 MHz, DMSO-d₆): δ = 3,00 (m, 1H); 3,60-3,70 (m, 4H); 3,90 (s, 3H); 6,50 (s, 1H); 7,70 (m, 5H).

### 5. Stufe: Darstellung von 2-(2,5-Diamino-4-methoxyphenyl)propane-1,3-diol, Dihydrochlorid

In eine Lösung von 14,6g (38,3mol) 4-({4-Amino-5-[2-hydroxy-1-(hydroxymethyl)ethyl]-2-methoxyphenyl}diazenyl)benzolsulfonsäure in 350mL Wasser, wurden bei Raumtemperatur 16,8g (107mmol) Natriumditionit eingetragen. Nach weiteren 45 Minuten rühren bei Raumtemperatur wurde das Reaktionsgemisch für 3 Stunden auf 80°C erhitzt. Danach wurde die erhaltene klare, gelbe Lösung auf Raumtemperatur abgekühlt und mit 130 mL verdünnter Salzsäure versetzt. Der resultierende Niederschlag wurde abfiltriert und die verbliebene Lösung am Rotationsverdampfer vollständig eingeengt. Der Rückstand wurde in 100mL Methanol aufgenommen, die unlöslichen Anteile abfiltriert und das Filtrat am Rotationsverdampfer vollständig eingeengt. Der verbliebene Feststoff wurde im Vakuum bei 40°C getrocknet.
Ausbeute: 8,1g = 100 % hell-beiger Feststoff
¹H-NMR (400 MHz, DMSO-d₆/D₂O): δ = 2,95 (m, 1H); 3,55 (m, 2H); 3,70 (m, 2H); 3,80 (s, 3H); 6,60 (s, 1H); 7,00 (s, 1H).

### Ausfärbungen

### Versuchsdurchführung

Für die Herstellung der Färbecreme wurden 50g einer Cremebasis in einem 250ml Becherglas eingewogen und bei 80°C geschmolzen. Die verwendete Cremebasis hatte die folgende Zusammensetzung:

| | |
|---|---|
| Hydrenol^{®} D¹ | 17,0 Gew.-% |
| Lorol^{®} tech.² | 4,0 Gew.-% |
| Texapon^{®} NSO³ | 40,0 Gew.-% |
| Dehyton^{®} K⁴ | 25,0 Gew.-% |
| Eumulgin^{®} B2⁵ | 1,5 Gew.-% |
| Wasser | 12,5 Gew.-% |

| | |
|---|---|
| ¹C₆₋₁₈-Fettalkohol (INCl-Bezeichnung: Cetearyl alcohol) (Cognis) ²C₁₂₋₁₈-Fettalkohol (INCl-Bezeichnung: Coconut alcohol) (Cognis) ³Laurylethersulfat, Natriumsalz (ca. 27,5% Aktivsubstanz; INCl-Bezeichnung: Sodium Laureth Sulfate) (Cognis) 4 N,N-Dimethyl-N-(C₈₋₁₈-kokosamidopropyl)ammoniumacetobetain (ca. 30% Aktivsubstanz; INCl-Bezeichnung: Aqua (Water), Cocamidopropyl Betaine) (Cognis) ⁵Cetylstearylalkohol mit ca. 20 EO-Einheiten (INCl-Bezeichnung: Ceteareth-20) (Cognis) | |

Es wurden jeweils 1/400 Mol der Entwickler- bzw. Kupplerkomponente getrennt in destilliertem Wasser suspendiert bzw. unter Erwärmen gelöst. Anschließend wurde Ammoniak (<1 ml; 25%ige Ammoniaklösung) zugegeben, bis der pH-Wert zwischen 9 und 10 lag.

Die gelösten Farbstoffvorprodukte wurden nacheinander in die heiße Creme eingearbeitet. Anschließend wurde mit destilliertem Wasser auf 97g aufgefüllt und mit Ammoniak ein pH-Wert von 9,5 eingestellt. Nach Auffüllen mit destilliertem Wasser auf 100 g wurde der Ansatz kaltgerührt (< 30°C), wobei eine homogene Creme entstand.

Für die Ausfärbungen wurden (soweit nichts anderes vermerkt ist) jeweils 25 g Färbecreme mit 25 g der folgenden Oxidationsmittelzubereitung vermischt.

| | |
|---|---|
| Dipicolinsäure | 0,1 Gew.-% |
| Natriumpyrophosphat | 0,03 Gew.-% |
| Turpinal^{®} SL⁶ | 1,50 Gew.-% |
| Texapon^{®} N28⁷ | 2,00 Gew.-% |
| Acrysol^{®} 22⁸ | 0,60 Gew.-% |
| Wasserstoffperoxid, 50 %ig | 12,0 Gew.-% |
| Natronlauge, 45%ig | 0,80 Gew.-% |
| Wasser | ad 100 |

| | |
|---|---|
| ⁶ 1-Hydroxyethan-1,1-diphosphonsäure (ca. 58 - 61% Aktivsubstanzgehalt; INCl-Bezeichnung: Etidronic Acid, Aqua (Water)) (Solutia) ⁷ Laurylethersulfat-Natrium-Salz (mind. 26,5 % Aktivsubstanzgehalt; INCl-Bezeichnung: Sodium Laureth Sulfate) (Cognis) ⁸ Acrylpolymer (ca. 29.5 - 30.5% Festkörper in Wasser; INCI-Bezeichnung: Acrylates/Steareth-20 Methacrylate Copolyme) In jede der so erhaltenen Mischungen wurde eine Haarsträhne (80 % ergraut; 330 mg bis 370 mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei 32 °C wurden die Haarsträhnen entnommen und mit einer wässrigen Texapon^{®} EVR-Lösung⁹ so oft gewaschen, bis der Farbüberschuß entfernt war. Die Haarsträhnen wurden an der Luft getrocknet, und ihr Farbton wurde unter der Tageslichtlampe (Farbprüfgerät HE240A) bestimmt und notiert (Taschenlexikon der Farben, A. Kornerup u. J.H. Wanscher, 3. unveränderte Auflage 1981, MUSTER-SCHMIDT Verlag; Zürich, Göttingen). ⁹ Laurylethersulfat-Natrium-Salz mit speziellen Zusätzen (ca. 34 bis 37% Aktivsubstanzgehalt; INCl-Bezeichnung: Sodium Lauryl Sulfate, Sodium Laureth Sulfate, Lauramide MIPA, Cocamide MEA, Glycol Stearate, Laureth-10) (Cognis) | |

Die bei den Ausfärbungs-Untersuchungen erhaltenen Ergebnisse sind in den nachstehenden Tabellen aufgeführt.

### Ausfärbungen mit den Beispielverbindungen

### 1. 2-[2,4-Diamino-3-(2-hydroxyethyl)phenyl]propan-1,3-diol, Dihydrochlorid

| Beispiel | Entwickler | Kuppler | erhaltene Nuance |
|---|---|---|---|
| 1 | p-Toluylendiamin | K1 | schwarzblau |
| 2 | 2,4,5,6-Tetraaminopyrimidin | K1 | oliv |
| 3 | p-Aminophenol | K1 | violettbraun |
| 4 | 2-(β-Hydroxyethyl)-p-phenylendiamin | K1 | dunkelblau |
| 5 | 4-Amino-3-methylphenol | K1 | rotbraun |
| 6 | Bis-(2-hydroxy-5-aminophenyl)methan | K1 | rotbraun |
| 7 | 4,5-Diamino-1-(2-hydroxyethyl)-pyrazol | K1 | dunkelmagenta |

| | | | |
|---|---|---|---|
| K1 : 2-[2,4-Diamino-3-(2-hydroxyethyl)phenyl]propan-1,3-diol, Dihydrochlorid | | | |

### II. 2-(2,4-Diamino-5-ethoxyphenyl)propan-1,3-diol, Dihydrochlorid

| Beispiel | Entwickler | Kuppler | erhaltene Nuance |
|---|---|---|---|
| 1 | p-Toluylendiamin | K2 | schwarzblau |
| 2 | 2,4,5,6-Tetraaminopyrimidin | K2 | fahl |
| 3 | p-Aminophenol | K2 | mattrot |
| 4 | 2-(ß-Hydroxyethyl)-p-phenylendiamin | K2 | schwarzblau |
| 5 | 4-Amino-3-methylphenol | K2 | nougatfarbig |
| 6 | Bis-(2-hydroxy-5-aminophenyl)methan | K2 | dunkelblond |
| 7 | 4,5-Diamino-1-(2-hydroxyethyl)-pyrazol | K2 | graurubin |

| | | | |
|---|---|---|---|
| K2 : 2-(2,4-Diamino-5-ethoxyphenyl)propan-1,3-diol, Dihydrochlorid, aus Beispiel | | | |

### III. 2-(2,4-Diamino-5-methoxyphenyl)propan-1,3-diol, Dihydrochlorid

| Beispiel | Entwickler | Kuppler | erhaltene Nuance |
|---|---|---|---|
| 1 | p-Toluylendiamin | K3 | schwarzblau |
| 2 | 2,4,5,6-Tetraaminopyrimidin | K3 | mattgrün |
| 3 | p-Aminophenol | K3 | violettbraun |
| 4 | 2-(ß-Hydroxyethyl)-p-phenylendiamin | K3 | schwarzblau |
| 5 | 4-Amino-3-methylphenol | K3 | violettbraun |
| 6 | Bis-(2-hydroxy-5-aminophenyl)methan | K3 | rotbraun |
| 7 | 4,5-Diamino-1-(2-hydroxyethyl)-pyrazol | K3 | rubin |

| | | | |
|---|---|---|---|
| K3: 2-(2,4-Diamino-5-methoxyphenyl)propan-1,3-diol, Dihydrochlorid, aus Beispiel III | | | |

### IV. 2-(2,5-Diamino-4-methoxyphenyl)propan-1,3-diol, Dihydrochlorid

| Beispiel | Kuppler | Entwickler | erhaltene Nuance |
|---|---|---|---|
| 1 | Resorcin | E1 | aubergine |
| 2 | 3-Amino-6-methoxy-2-methylaminopyridin | E1 | schiefergrau |
| 3 | 2,4-Diaminophenoxyethanol | E1 | schwarzblau |
| 4 | 4-Amino-2-hydroxytoluol | E1 | dunkelviolett |

| | | | |
|---|---|---|---|
| E1: 2-(2,5-Diamino-4-methoxyphenyl)propan-1,3-diol, Dihydrochlorid, aus Beispiel IV | | | |

## Patentansprüche

1. Mittel zur Färbung keratinischer Fasern, insbesondere menschlicher Haare, enthaltend in einem kosmetisch akzeptablen Träger als Farbstoffvorprodukt mindestens ein Phenylpropandiol-Derivat der allgemeinen Formel (I) 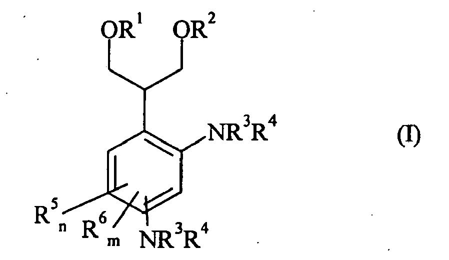 mit der Bedeutung
R¹, R² R³ R⁴ unabhängig voneinander H, C₁₋₆-Alkyl, C₁₋₆-Hydroxyalkyl, C₂₋₆- Polyhydroxyalkyl, C₇₋₁₂Arylalkyl, C₂₋₆-Alkoxyalkyl, C₁₋₆- Aminoalkyl, C₂₋₁₀-Alkylaminoalkyl, C₃₋₁₂-Dialkylaminoalkyl, wobei R¹ und R² auch gemeinsam einen C₁₋₃-Alkylenrest bilden können, der durch Halogen, Hydroxy oder C₁₋₄-Alkyl substituiert sein kann, Halogenalkyl, gegebenenfalls substituierter aromatischer Rest, C₂₋₆-Alkenyl,
R⁵ unabhängig voneinander H, OH, Halogen, NH₂, C₁₋₆-Alkylamino, C₂₋₁₂-Dialkylamino, NO₂, COOH, CN, C₁₋₆-Alkyl, C₁₋₆-Hydroxyalkyl, C₂₋₆-Polyhydroxyalkyl, C₇₋₂-Arylalkyl, C₂₋₆-Alkoxyalkyl, C₁₋₆- Aminoalkyl, C₂₋₁₀-Alkylaminoalkyl, C₃₋₁₂-Dialkylaminoalkyl, C₁₋₆- Alkoxy, gegebenenfalls substituierter aromatischer Rest, C₂₋₆- Alkenyl,
R⁶ H, Halogen, C₁₋₆-Alkoxy, C₁₋₆-Alkylthio,
m 0 oder 1,
n ganze Zahl von 0 bis (3-m),
ausgewählt aus Phenylpropandiol-Derivaten der allgemeinen Formeln (Ia) bis (Id)

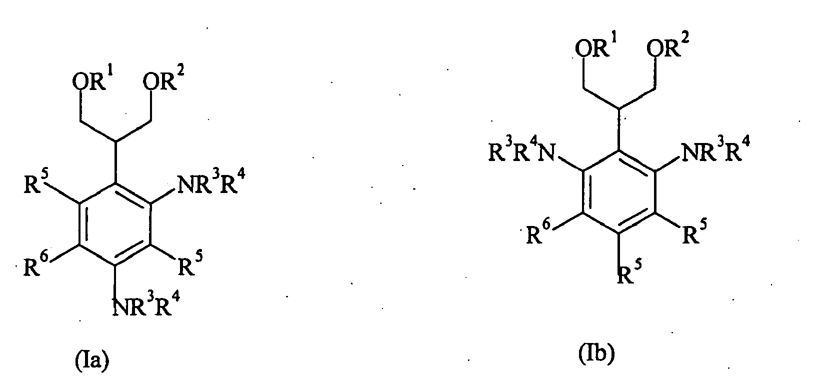

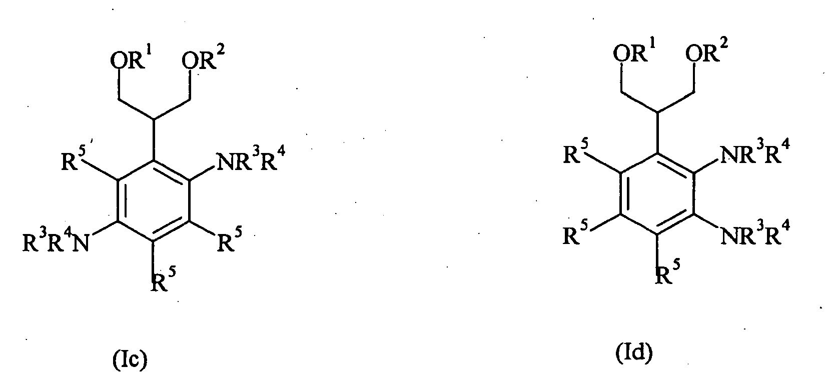

2. Mittel nach Anspruch 1, **dadurch gekennzeichnet, dass** im Derivat der allgemeinen Formel (I) R¹, R², R³, R⁴ unabhängig voneinander H, C₁₋₄-Alkyl oder C₁₋₄-Hydroxylalkyl bedeuten.

3. Mittel nach Anspruch 2, **dadurch gekennzeichnet, dass** im Derivat der allgemeinen Formel (I) R¹ und R² Wasserstoff und R³ und R⁴ Wasserstoff oder C₁₋₄-Hydroxyalkyl bedeuten.

4. Mittel nach Anspruch 3, **dadurch gekennzeichnet, dass** im Derivat der allgemeinen Formel (I) R¹ bis R⁴ Wasserstoff bedeuten.

5. Mittel nach einem der Ansprüche 1 bis 4, **dadurch gekennzeichnet, dass** im Derivat der allgemeinen Formel (I) R⁵ unabhängig voneinander Wasserstoff, C₁₋₄-Alkyl oder C₁₋₄-Hydroxyalkyl bedeutet.

6. Mittel nach Anspruch 5, **dadurch gekennzeichnet, dass** im Derivat der allgemeinen Formel (I) m 0 oder 1 bedeutet.

7. Mittel nach Anspruch 6, **dadurch gekennzeichnet, dass** im Derivat der allgemeinen Formel (I) R⁶ Wasserstoff oder C₁₋₄-Alkoxy bedeutet.

8. Mittel nach einem der Ansprüche 1 bis 7, **dadurch gekennzeichnet, dass** das Phenylpropandiol-Derivat die allgemeine Formel (Ie) aufweist

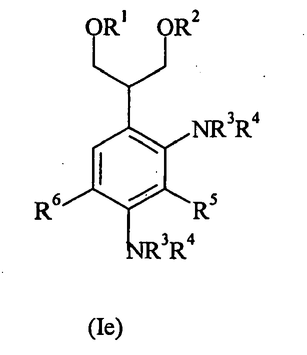

9. Mittel nach einem der Ansprüche 1 bis 8, **dadurch gekennzeichnet, dass** es mindestens eine (weitere) Entwicklerkomponente enthält.

10. Mittel nach einem der Ansprüche 1 bis 9, **dadurch gekennzeichnet, dass** es mindestens eine (weitere) Kupplerkomponente enthält.

11. Mittel nach einem der Ansprüche 1 bis 10, **dadurch gekennzeichnet, dass** es weiterhin mindestens einen direktziehenden Farbstoff enthält.

12. Mittel nach Anspruch 11, **dadurch gekennzeichnet, dass** der direktziehende Farbstoff kationisch ist.

13. Verfahren zur Färbung keratinischer Fasern, bei dem ein Mittel nach einem der Ansprüche 1 bis 12 auf die Fasern aufgetragen wird und nach einer Einwirkzeit wieder abgespült wird.

14. Verwendung von Phenylpropandiol-Derivaten der allgemeinen Formel (I), wie sie in einem der Ansprüche 1 bis 8 definiert sind, zur Färbung keratinischer Fasern, insbesondere menschlicher Haare.

15. Synthesezwischenstufe, ausgewählt aus der Gruppe, gebildet von 2-(5-Ethoxy-2,4-dinitrophenyl)propan-1,3-diol, 2-(5-Methoxy-2,4-dinitrophenyl)propan-1,3-diol, 2-(3-(2-Hydroxyethyl)-2,4-dinitrophenyl]propan-1,3-diol.

## Claims

1. An agent for dyeing keratinic fibers, in particular human hair, containing in a cosmetically acceptable carrier, as a dye precursor product, at least one phenylpropanediol derivative of general formula (I) 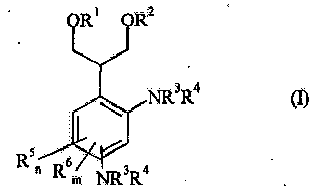 with the meaning:
R¹, R², R³, R⁴ represent independently of each other H, C₁-C₆ alkyl, C₁-C₆ hydroxyalkyl, C₂-C₆ polyhydroxyalkyl, C₇-C₁₂ arylalkyl, C₂-C₆ alkoxyalkyl, C₁-C₆ aminoalkyl, C₂-C₁₀ alkylaminoalkyl, C₃-C₁₂ dialkylaminoalkyl, wherein R¹ and R² may also form together a C₁- C₃ alkylene radical, which may be substituted by a halogen, hydroxy or C₁-C₄ alkyl, haloalkyl, an optionally substituted aromatic radical, C₂-C₆ alkenyl,
R⁵ represents independently of each other, H, OH, a halogen, NH₂, C₁-C₆ alkylamino, C₂-C₁₂ dialkylamino, NO₂, COOH, CN, C₁-C₆ alkyl, C₁-C₆ hydroxyalkyl, C₂-C₆ polyhydroxyalkyl, C₇-C₁₂ arylalkyl, C₂-C₆ alkoxyalkyl, C₁-C₆ aminoalkyl, C₂-C₁₀ alkylaminoalkyl, C₃-C₁₂ dialkylaminoalkyl, C₁-C₆ alkoxy, an optionally substituted aromatic radical, C₂-C₆ alkenyl,
R⁶ represents H, a halogen, C₁-C₆ alkoxy, C₁-C₆ alkylthio,
m represents 0 or 1,
n represents an integer from 0 to (3-m),
selected from phenylpropanediol derivatives of general formulae (Ia)-(Id)

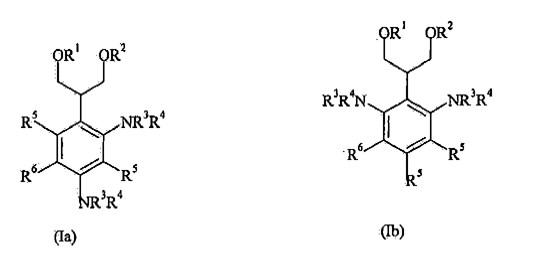

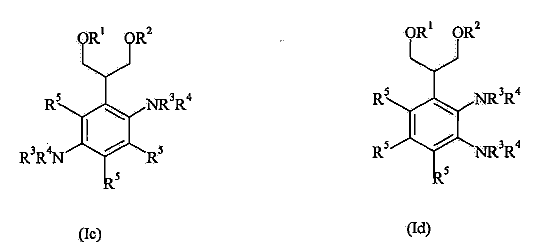

2. The agent according to claim 1, **characterized in that** in the derivative of general formula (I), R¹, R², R³, R⁴ represent independently of each other, H, C₁-C₄ alkyl or C₁-C₄ hydroxyalkyl.

3. The agent according to claim 2, **characterized in that** in the derivative of general formula (I), R¹ and R² represent a hydrogen and R³ and R⁴, a hydrogen or C₁-C₄ hydroxyalkyl.

4. The agent according to claim 3, **characterized in that** in the derivative of general formula (I), R¹-R⁴ represent a hydrogen.

5. The agent according to any of claims 1 to 4 **characterized in that**, in the derivative of general formula (I), R⁵ represents independently of each other a hydrogen, C₁-C₄ alkyl, or C₁-C₄ hydroxyalkyl.

6. The agent according to claim 5, **characterized in that** in the derivative of general formula (I), m represents 0 or 1.

7. The agent according to claim 6, **characterized in that** in the derivative of general formula (I), R⁶ represents a hydrogen or a C₁-C₄ alkoxy.

8. The agent according to any of claims 1 to 7, **characterized in that** the phenylpropanediol derivative has the general formula (le)

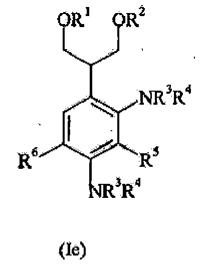

9. The agent according to any of claims 1 to 8, **characterized in that** it contains at least one (further) developer component.

10. The agent according to any of claims 1 to 9, **characterized in that** it contains at least one (further) coupler component.

11. The agent according to any of claims 1 to 10, **characterized in that** it further contains at least one substantive dye.

12. The agent according to claim 11, **characterized in that** the substantive dye is cationic.

13. A method for dyeing keratinic fibers, in which an agent according to any of claims 1 to 12 is applied on the fibers and after an exposure time is again rinsed.

14. The use of phenylpropanediol derivatives of general formula (I), as they are defined in any of claims 1 to 8, for dyeing keratinic fibers, in particular human hair.

15. A synthesis intermediate, selected from the group formed by 2-(5-ethoxy-2,4-dinitrophenyl)propane-1,3-diol, 2-(5-methoxy-2,4-dinitro-phenyl)-propane-1,3-diol, 2-[3-(2-hydroxyethyl)-2,4-dinitrophenyl]propane-1,3-diol.

## Revendications

1. Agent pour la teinture de fibres kératiniques, en particulier de cheveux humains, contenant, dans un support acceptable du point de vue cosmétique, à titre de précurseur de colorant, au moins un dérivé de phénylpropanediol répondant à la formule générale (I) 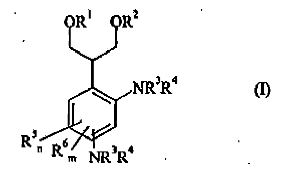 dans laquelle
R¹, R², R³, R⁴ représentent, indépendamment l'un de l'autre, un atome d'hydrogène, un groupe alkyle en C₁-C₆, un groupe hydroxyalkyle en C₁-C₆, un groupe polyhydroxyalkyle en C₂-C₆, un groupe arylalkyle en C₇-C₁₂, un groupe alcoxyalkyle en C₂-C₆, un groupe aminoalkyle en C₁-C₆, un groupe alkylaminoalkyle en C₂-C₁₀, un groupe dialkylaminoalkyle en C₃-C₁₂, R¹ et R² pouvant également former ensemble un radical alkylène en C₁-C₃ qui peut être substitué par un atome d'halogène, par un groupe hydroxyle ou par un groupe alkyle en C₁-C₄, un groupe halogénoalkyle, un radical aromatique éventuellement substitué, un groupe alcényle en C₂-C₆ ;
R⁵ représente, de manière indépendante, un atome d'hydrogène, un groupe OH, un atome d'halogène, un groupe NH₂, un groupe alkyl(en C₁-C₆)amino, un groupe dialkyl(en C₂-C₁₂)amino, un groupe NO₂, un groupe COOH, un groupe CN, un groupe alkyle en C₁-C₆, un groupe hydroxyalkyle en C₁-C₆, un groupe polyhydroxyalkyle en C₂-C₆, un groupe arylalkyle en C₇-C₁₂, un groupe alcoxyalkyle en C₂-C₆, un groupe aminoalkyle en C₁-C₆, un groupe alkylaminoalkyle en C₂-C₁₀, un groupe dialkylaminoalkyle en C₃-C₁₂, un groupe alcoxy en C₁-C₆, un radical aromatique éventuellement substitué, un groupe alcényle en C₂-C₆ ;
R⁶ représente un atome d'hydrogène, un atome d'halogène, un groupe alcoxy en C₁-C₆, un groupe alkyl(en C₁-C₆)thio ;
m est égal à 0 ou 1 ;
n représente un nombre entier de (0 à 3-m)
choisi parmi des dérivés de phénylpropanediol répondant aux formules générales (Ia) à (Id)

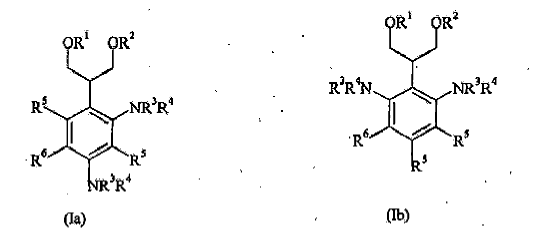

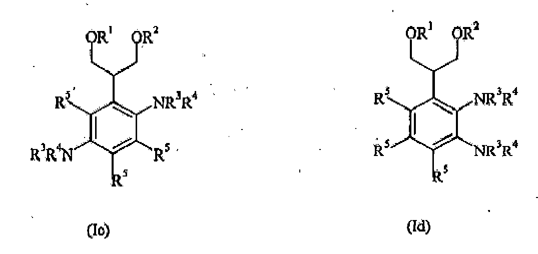

2. Agent selon la revendication 1, **caractérisé en ce que**, dans le dérivé répondant à la formule générale (I), R¹, R², R³, R⁴ représentent, indépendamment l'un de l'autre, un atome d'hydrogène, un groupe alkyle en C₁-C₄ ou un groupe hydroxyalkyle en C₁-C₄.

3. Agent selon la revendication 2, **caractérisé en ce que**, dans le dérivé répondant à la formule générale (I), R¹ et R² représentent un atome d'hydrogène et R³ et R⁴ représentent un atome d'hydrogène ou un groupe hydroxyalkyle en C₁-C₄.

4. Agent selon la revendication 3, **caractérisé en ce que**, dans le dérivé répondant à la formule générale (I), R¹ à R⁴ représentent un atome d'hydrogène.

5. Agent selon l'une quelconque des revendications 1 à 4, **caractérisé en ce que**, dans le dérivé répondant à la formule générale (I), R⁵ représente, de manière indépendante, un atome d'hydrogène, un groupe alkyle en C₁-C₄ ou un groupe hydroxyalkyle en C₁-C₄.

6. Agent selon la revendication 5, **caractérisé en ce que**, dans le dérivé répondant à la formule générale (I), m est égal à 0 ou 1.

7. Agent selon la revendication 6, **caractérisé en ce que**, dans le dérivé répondant à la formule générale (I), R⁶ représente un atome d'hydrogène ou un groupe alcoxy en C₁-C₄.

8. Agent selon l'une quelconque des revendications 1 à 7, **caractérisé en ce que** le dérivé de phénylpropanediol présente la formule générale (le)

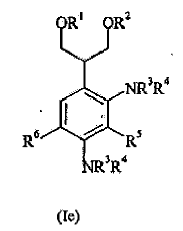

9. Agent selon l'une quelconque des revendications 1 à 8, **caractérisé en ce qu'**il contient au moins un composant développeur (supplémentaire).

10. Agent selon l'une quelconque des revendications 1 à 9, **caractérisé en ce qu'**il contient au moins un composant coupleur (supplémentaire).

11. Agent selon l'une quelconque des revendications 1 à 10, **caractérisé en ce qu'**il contient en outre au moins un colorant direct.

12. Agent selon la revendication 11, **caractérisé en ce que** le colorant direct est de type cationique.

13. Procédé pour la teinture de fibres kératiniques, dans lequel on applique sur les fibres un agent selon l'une quelconque des revendications 1 à 12 et on l'en élimine par rinçage après avoir laissé agir pendant un certain temps.

14. Utilisation de dérivés de phénylpropanediol répondant à la formule générale (I), tels qu'ils ont été définis dans l'une quelconque des revendications 1 à 8, pour la teinture de fibres kératiniques, en particulier de cheveux humains.

15. Produit intermédiaire de synthèse, choisi parmi le groupe formé par le 2-(5-éthoxy-2,4-dinitrophényl)propane-1,3-diol, le 2-(5-méthoxy-2,4-dinitrophényl)propane-1,3-diol, le 2-[3-(2-hydroxyéthyl)-2,4-dinitrophényl]-propane-1,3-diol.
